# EUROPEAN PATENT APPLICATION

(11) **EP 1 454 577 A2**
(43) Date of publication of application: **08.09.2004**
(21) Application number: 04251231.9
(22) Date of filing: 03.03.2004
(51) Int. Cl.: A47K 7/03, A61K 7/50

(54) **Disposable skin cleansing implement**

(30) Priority: 04.03.2003 US 379074
(71) Applicant: Johnson & Johnson Consumer Companies, Inc., Skillman, New Jersey 08558-9418 (US)
(72) Inventor: Siegwart, Kathleen Ann, Milford, NJ 08848 (US)
(74) Representative: Mercer, Christopher Paul

(57) **Abstract**

A dry, disposable cleansing implement including: an inner layer; and an outer exfoliating layer, wherein the outer layer substantially surrounds the inner layer, the inner layer is impregnated with a cleansing composition, and the inner layer and outer layer are held together by securing means is disclosed.

## Description

### Background Of The Invention

The present invention relates to a skin cleansing implement. The skin cleansing implement combines a material that is useful for scrubbing or exfoliating the skin with a material that is useful for cleansing the skin. The implement is constructed of an outer layer made of open mesh or apertured film and an inner layer made of a material that is impregnated with a cleansing solution.

Many consumers achieve personal cleansing by using a bar of soap as a cleansing material and an implement, such as a washcloth, a sponge, or the like to apply the soap to the body. Recently, liquid cleansers have become increasingly more prevalent. The liquid cleanser is typically applied to the body with an implement, such as a washcloth, a sponge, a puff/pouf and the like. Gathered implements, such as puffs/poufs have gained in popularity due to their ability to provide cleansing and exfoliation benefits.

One problem associated with cleansing implements is that they require the addition of a cleansing material, such as soap, a liquid cleanser, or a gel. Having separate cleansing implements and cleansing materials takes up more space in the shower and is inconvenient. A second problem associated with the use of cleansing implements concerns personal hygiene and other sanitary issues. The implements may support microbial growth over time. This may result in bacteria and fungi being applied to the skin.

Most recently, personal cleansing wipes or cloths have gained popularity for use as facial cleansers/exfoliators, skin care implements, and the like. These articles, however, are flat, two-dimensional structures that do not lend themselves to providing optimal cleansing and/or exfoliation, ease of use, or like benefits.

There is a need for a cleansing implement that contains a dry cleanser, provides skin exfoliation, and is disposable.

United States Patent No. 5,727,278 describes a cleansing device that is a reticulated polyurethane sponge. Since these sponges are not disposable, i.e., designed to be disposed of in one week or less, over time the sponges may retain moisture and promote microbial growth.

United States Patent Nos 6,092,257 and 6,038,727 describe cleansing devices. The devices have various textures and fibers to produce a multi-layer ruffled body forming a "bath ball". The bath ball has naturally spread dense ruffles, enhancing soap foaming and cleansing capabilities. The soap component is taught to be applied separately to the bath ball.

United States Patent 6,015,242 describes a body cleansing puff which contains pieces of solid soap. The device is filled with solid soap and re-filled as the solid soap is used.

United States Patent 6,368,003 describes a hand-held washing device that contains soap within the interior. The soap is contained within a reservoir and dispensed via a nozzle. The soap may be in a variety of forms including bar and fluid. The device is re-filled as the soap is used.

European Patent Application No. EP 1125540 describes a textured film cleansing device made from at least one layer of gathered textured film. The device produces excellent lather.

United States Patent 6,063,397 describes dry wipes that are useful for personal cleansing.

Despite the disclosure of the references, there is a continuing need for a cleansing implement that contains a dry cleanser, provides skin exfoliation, and is disposable.

### Summary Of The Invention

The present invention provides a dry, disposable cleansing implement having an inner layer; and an outer exfoliating layer, wherein the outer layer substantially surrounds the inner layer, the inner layer is impregnated with a cleansing composition, and the inner layer and outer layer are held together by securing means.

### Detailed Description Of The Invention

The cleansing implement of the present invention has an inner layer. Suitable materials for the inner layer are known in the art of wipes and include, but are not limited to, a woven fabric, a knit fabric, a nonwoven fabric, a laminate of a fabric and a polymeric film, such as a polyolefin film, a flocked fabric, and combinations thereof. Methods of making woven and knit cloths are not a part of this invention and, being well known in the art, are not described in detail herein.

One type of nonwoven cloth substrate utilized in the present invention is made by air- or water-laying processes in which the fibers or filaments are first cut to desired lengths from long strands, passed into a water or air stream, and then deposited onto a screen through which the fiber-laden air or water is passed. The deposited fibers or filaments are then adhesively bonded together, and otherwise treated as desired to form the woven, nonwoven, or cellulose cloth.

The inner layer utilized in the present invention may be a thermal bonded nonwoven cloth (whether or not resin-containing) which can be made of polyesters, polyamides, polyolefins, or other thermoplastic fibers which can be spun bonded, i.e., the fibers are spun out onto a flat surface and bonded (melted) together by heat or chemical reactions.

When nonwovens are utilized, the nonwoven cloth substrates are generally adhesively bonded fibers or filamentous products having a web or carded fiber structure (when the fiber strength is suitable to allow carding) or comprising fibrous mats in which the fibers or filaments are distributed haphazardly or in random array (i.e., an array of fibers in a carded web where partial orientation of the fibers is frequently present, as well as a completely haphazard distributional orientation), or substantially aligned. The fibers or filaments can be natural (e.g., wool, silk, jute, hemp, cotton, linen, sisal, or ramie) or synthetic (e.g., rayon, cellulose ester, polyvinyl derivatives, polyolefins, such as polyethylene and polypropylene, polyamides, such as nylon 6, nylon 6,6, or polyesters, such as polyethylene terephthalate and polybutylene terephthalate), or combinations thereof. These nonwoven materials are generally described in the INDA "NONWOVEN FABRICS HANDBOOK", (1999), hereby incorporated by reference for nonwoven substrates and their methods of manufacture.

The basis weight of the inner layer may vary, but generally ranges from about 20 grams per square meter to about 500 grams per square meter, for example from about 50 grams per square meter to about 150 grams per square meter.

The implement of the present invention has an outer exfoliating layer.

Materials that are useful for exfoliating the skin are known in the art. Suitable materials include, but are not limited to, apertured films and open-mesh netting. Open-mesh netting is preferred and is typically made from a molten polymer that is extruded onto a die that creates holes in the polymer. The polymer is then cooled into a film that has holes in it. The holes may be in the shape of a diamond, i.e., diamond mesh scrim, a square, a hexagon or other shapes known in the art.

Diamond mesh scrim is useful as an exfoliating layer. Diamond mesh scrim is a plastic netting made by an extrusion process using counter-rotating die heads, each of which has multiple extrusion orifices located at the edge of each die. The counter rotation of the die heads causes extruded filaments or strands to align in two directions at angles to the machine direction of the extruded tubing. The strands periodically intersect to form nodes. The two strand directions are typically at acute angles to each other, such that strands form diamond patterns with nodes at each comer. United States Patent No. 3,957,565 describes this process in greater detail and is hereby incorporated by reference.

The outer exfoliating layer may be made of any suitable material capable of providing exfoliating benefits to the skin. Suitable materials include, but are not limited to, polyolefins, such as polyethylene, low density polyethylene, linear low density polyethylene, high density polyethylene, and polypropylene, polyesters, and ethylene vinyl acetate. Low density polyethylene is preferred.

The cleansing implement includes a securing means for substantially permanently holding the inner layer and the outer layer together. As used herein, "substantially permanently" means a period of time at least as long as the implement is suitable for cleansing uses.

Examples of suitable securing methods include heat sealing with a sealer capable of reaching a temperature greater than the melting temperature of the outer layer; ultrasonic sealing; pressure sealing; tying with a ribbon, cord, strip, string such as a band, and the like, see United States Patent No. 5,727,278, which is hereby incorporated by reference; applying hooks and loops such as that registered as "VELCRO", adhesive, elastic, tape such as double-sided adhesive tape, heat shrinkable film, or another known fastening device thereto; applying a locking tether thereto, see U.S. Patent No. 5,784,747, which is hereby incorporated by reference, and the like. Preferably the securing means is comprised of a tying or otherwise wrapping a cord, which is either elastic or inelastic, around the gathered arrangement as well as applying an adhesive-coated cord to the stretched state of the outer layer. In the latter embodiment, the outer layer becomes bunched together after it is released from its stretched state.

In embodiments wherein the securing means is a string, ribbon, or cord, the string may be made from any suitable material, such as a separate strip of mesh netting, perforated film itself, nylon, or cotton. The string may be wound about a circumferential portion of the implement, preferably centrally located, then drawn inwardly toward the center of the implement and secured to itself to form a winding of the string with a substantially smaller circumference than that of the implement.

In this embodiment, the securing means not only holds the inner layer and outer layer together, but also, serves to gather the inner and outer layer. By "gathered," it is meant to fold, pleat, smock, for example, in a corrugated manner, or any other known technique for pulling the cleansing implement together into a desired shape. By winding the string, or applying any other suitable securing means, about the implement, the interior portions of the ruffles then become gathered together toward the center of the implement. As a result of this gathering effect, a central portion is formed. A pair of lobes formed of the loop ends of the ruffles, which extend radially in every direction, project outwardly from the central portion. See United States Patent No. 5,784,747, which is hereby incorporated by reference. Because each loop end generally has the same length, the lobes of the implement formed thereby generally have a sphere-like shape. Due to the general pliability of the outer layer material that makes up the implement, the spherical shape of the implement is deformable during use of the cleansing implement, e.g. the application of pressure and contacting of the implement to the desired surface to be cleaned in a scrubbing or scouring motion. Generally, the sphere-like shape of the implement will return after use without becoming separated.

The inner layer is impregnated with a cleansing composition by means known in the art. Suitable cleansing compositions generally include one or more surfactants. Typically, a lathering surfactant is included in the cleansing composition. What is meant by a lathering surfactant is a surfactant that generates lather when combined with water and mechanically agitated. Examples of lathering surfactants include, but are not limited to, anionic, nonionic, cationic, and amphoteric lathering surfactants.

Non-limiting examples of anionic lathering surfactants include those selected from the group consisting of sarcosinates, sulfates, isethionates, taurates, phosphates, lactylates, and glutamates. Specific examples include, but are not limited to, those selected from the group consisting of sodium lauryl sulfate, ammonium lauryl sulfate, ammonium laureth sulfate, sodium laureth sulfate, sodium trideceth sulfate, ammonium cetyl sulfate, sodium cetyl sulfate, ammonium cocoyl isethionate, sodium lauroyl isethionate, sodium lauroyl lactylate, triethanolamine lauroyl lactylate, sodium caproyl lactylate, sodium lauroyl sarcosinate, sodium myristoyl sarcosinate, sodium cocoyl sarcosinate, sodium lauroyl methyl taurate, sodium cocoyl methyl taurate, sodium lauroyl glutamate, sodium myristoyl glutamate, and sodium cocoyl glutamate and mixtures thereof.

Non-limiting examples of nonionic lathering surfactants include those selected from the group consisting of alkyl glucosides, alkyl polyglucosides, polyhydroxy fatty acid amides, alkoxylated fatty acid esters, lathering sucrose esters, amine oxides, and mixtures thereof. Specific examples include, but are not limited to, nonionic surfactants to those selected form the group consisting of C8-C14 glucose amides, C8- C14 alkyl polyglucosides, sucrose cocoate, sucrose laurate, lauramine oxide, cocoamine oxide, and mixtures thereof.

Non-limiting examples of amphoteric lathering surfactants, which also includes zwitterionic lathering surfactants, are those selected from the group consisting of betaines, sultaines, hydroxysultaines, alkyliminoacetates, iminodialkanoates, aminoalkanoates, and mixtures thereof.

Non-limiting examples of amphoteric surfactants of the present invention include disodium lauroamphodiacetate, sodium lauroamphoacetate, cetyl dimethyl betaine, cocoamidopropyl betaine, cocoamidopropyl hydroxy sultaine, and mixtures thereof.

Skin care actives, moisturizers, and the like may also be impregnated into the implements of the present invention. The compositions may be loaded onto the inner layer by dipping the inner layer in the composition, spraying the composition onto the inner layer, and other means known in the art. The inner layer may be dried through the use of heating equipment or vacuum driers to provide dry implements. Alternatively, a cleansing or skin care formulation may be applied in the form of a concentrate to the inner layer to provide dry implements.

The cleansing implements are sold dry. The consumer wets the implement with water when ready for use. As used herein, "dry" means that the cleansing implement contains less than about 15 percent by weight, preferably less than about 10 percent by weight water, based on the total weight of the cleansing implement.

Although the inner layer and the outer layer are secured together, they remain separate layers. In other words, the inner layer and the outer layer are not laminated together. It is believed that by keeping inner layer and outer layer separated, the implement is capable of providing improved lathering properties.

The articles of the invention are disposable. As used herein, disposable means that the article is utilized for about one week or less, and then disposed of. When the cleansing composition has been used up, the cleansing implement may be disposed of. Alternatively, a separate solid cleanser or a liquid cleanser may be used with the cleansing implement until the cleansing implement is disposed of.

The following examples are provided for illustrative purposes. The invention should not be construed as being limited to the details thereof.

### Example 1

A 3 foot section of outer layer material was cut from a roll of tubular low density polyethylene diamond mesh netting (from DelStar Technologies, Inc.). A Johnson's ® Gentle Cleansing Cloth (made of polyester nonwoven having a basis weight of 75 grams per square meter commercially available Johnson & Johnson) was cut into two 8 inch x 4 inch sections to be used as the inner layer of the cleansing implement. The two sections of cleansing cloths were placed edge to edge within the section of diamond mesh netting. The netting and cloth were gathered in a corrugated manner and the middle of the gathered article was secured by tying a 10 inch piece of nylon needle craft yam around the implement and into a knot.

The resulting disposable cleansing implement provided exfoliating and cleansing benefits to the skin.

## Claims

1. A dry, disposable cleansing implement comprising:
an inner layer; and
an outer exfoliating layer,
wherein the outer layer substantially surrounds the inner layer, the inner layer is impregnated with a cleansing composition, and the inner layer and outer layer are held together by securing means.

2. The cleansing implement according to claim 1 wherein the inner layer is a woven fabric, a knit fabric, a non-woven fabric, a laminate of a fabric and a polymeric film, a flocked fabric or a combination thereof.

3. The cleansing implement according to claim 1 or claim 2 wherein the outer layer is an apertured film or an open-mesh netting.

4. The cleansing implement according to any one of claims 1 to 3 wherein securing means is heat sealing, ultrasonic sealing, pressure sealing or tying.

5. The cleansing implement according to any one of claims 1 to 4 wherein the implement contains less than about 15 percent by weight water, based on the total weight of the cleansing implement.

6. The cleansing implement according to claim 5 wherein the implement contains less than about 10 percent by weight water, based on the total weight of the cleansing implement.

7. The cleansing implement according to any one of claims 1 to 6 wherein the cleansing implement is gathered.

8. The cleansing implement according to claim 7 wherein the gathering is folding, pleating or smocking.

9. The cleansing implement according to any one of claims 1 to 8 wherein the cleansing composition further includes a skin care active ingredient.

10. The cleansing implement according to claim 9 wherein the skin care active ingredient is a moisturizer.
